# EUROPEAN PATENT APPLICATION

(11) **EP 0 613 669 A2**
(43) Date of publication of application: **07.09.1994**
(21) Application number: 94301406.8
(22) Date of filing: 28.02.1994
(51) Int. Cl.: A61F 13/02

(54) **Process for making an adhesive dressing**

(30) Priority: 01.03.1993 ZA 931441
(71) Applicant: JOHNSON & JOHNSON CONSUMER PRODUCTS, INC., Skillman, New Jersey 08558 (US)
(72) Inventor: Sperinck, Peter George, Randburg, Transvaal Province (ZA); Charlton, Douglas John, Randburg, Transvaal Province (ZA)
(74) Representative: Fisher, Adrian John

(57) **Abstract**

A process for making an adhesive dressing comprises continuously feeding a strip (24) having at least portions which are absorbent, through an adhesive application zone (40). An adhesive is applied to one side of the strip in such a fashion that the adhesive coated strip has at least one absorbent pad area (30). The adhesive coated strip is fed directly and continuously to a release paper application zone (60) in which release paper is applied over the adhesive adjacent the absorbent pad area, to form a continuous adhesive dressing.

## Description

THIS INVENTION relates to a process for making an adhesive dressing.

According to the invention, there is provided a process for making an adhesive dressing, which comprises
continuously feeding a strip having at least portions which are absorbent, through an adhesive application zone;
applying an adhesive to one side of the strip in such a fashion that the adhesive coated strip has at least one absorbent pad area; and
feeding the adhesive coated strip directly and continuously to a release paper application zone in which release paper is applied over the adhesive adjacent the absorbent pad area, to form a continuous adhesive dressing.

While in principle a strip having only portions which are absorbent can be used, it is envisaged that the entire strip will normally be absorbent.

The process of the invention is characterized thereby that it is continuous, ie the strip passes continuously and directly from one zone to the next, without intermediate taking up thereof into a roll.

The process is also characterized thereby that separate absorbent pads are not applied to the strip; the adhesive is applied in such a fashion that the adhesive coated strip has said at least one absorbent pad area which becomes the absorbent pad of individual dressings once the continuous dressing has been cut into individual dressings, as hereinafter described.

Thus, the adhesive may be applied intermittently so that the absorbent pad area is defined by non-adhesive coated areas or portion of the strip. Instead, the adhesive may be coated continuously over the whole strip, so that the absorbent pad area is also coated with adhesive; however, the adhesive is then such that this zone will not adhere to a moist wound in use and will allow fluid absorbency, while the adhesive adjacent the absorbent pad area will adhere to dry skin adjacent such a moist wound, to secure the dressing in position. Examples of such adhesives are emulsion and hot melt acrylic adhesives which do not adhere to moist surfaces.

The adhesive may be a pressure sensitive adhesive. It may be a curable adhesive such as a solvent based adhesive or an emulsion adhesive requiring only drying. Instead, however, it may be a hot melt adhesive.

Thus, a single absorbent pad area, eg an absorbent pad area extending along the strip, may be provided. Instead, a plurality of the absorbent pad areas spaced longitudinally or laterally on the strip, may be provided.

The absorbent strip may comprise a backing layer having an absorbent layer thereon. The process may then include passing the backing layer continuously through an absorbent strip forming zone ahead of the adhesive application zone, and applying the absorbent layer continuously to the backing layer in the strip forming zone. The backing layer may then be cellulose based, may be in the form of a polymeric film, may be in the form of either a knitted or a non-woven fabric, may be of stretch or non-stretch form, or may be combinations thereof.

The absorbent layer may, in one version of the invention, be an absorbent fabric. However, in another version of the invention, it may be of curable foam material. The process may then include passing the strip from the strip forming zone through a curing zone ahead of the adhesive application zone, for curing of the foam material.

Instead, however, the absorbent strip may be characterized by the absence of a backing layer. The strip can then be of any suitable absorbent material, but Applicant believes that an absorbent foamed material will give good results.

The process may then include forming the absorbent foamed strip continuously in a strip forming zone ahead of the adhesive application zone. The process may also include passing the foamed strip from the strip forming zone through a curing zone where the foam is cured and stabilized, before passing it to the adhesive application zone. However, when the adhesive is a curable adhesive, the process may include passing the adhesive coated strip through a further curing zone located after the adhesive application zone and in which the adhesive is cured. Instead, a single curing zone, located after the adhesive application zone and in which curing of both the foam and adhesive is effected, may be provided.

The process may include passing the continuous adhesive dressing from the release paper application zone to a cutting zone, and cutting it into individual dressings of a desired shape and size in the cutting zone. The cutting may be effected by means of a die cutting arrangement, eg by cutting a plurality of dressings side-by-side from the strip.

The foam material may be SBR styrene butadiene rubber or a polyurethane foam. However, any other foam which can be formed into a relatively thin layer can be used. The foam material may contain absorbency enhancing materials, such as acrylic superabsorbent powder. It may also contain a medication such as silver sulphur diazine and/or a facing layer such as a plastic net or a non-woven fabric.

The pad area can thus be continuous, ie extending along the strip, or spaced pad areas, ie spaced laterally and/or longitudinally apart, can be provided

The invention will now be described by way of example with reference to the accompanying diagrammatic drawings.

### In the drawings,

FIGURE 1 shows a simplified flow diagram of a process for making an adhesive dressing, according to one embodiment of the invention;
FIGURE 2 shows, in part, a sectional view through II-II in Figure 1;
FIGURE 3 shows a plan view of a cutting die for effecting the cutting operation in Figure 1;
FIGURE 4 shows a plan view of an adhesive dressing formed by the process of Figure 1; and
FIGURE 5 shows a simplified flow diagram of a process for making an adhesive dressing, according to another embodiment of the invention.

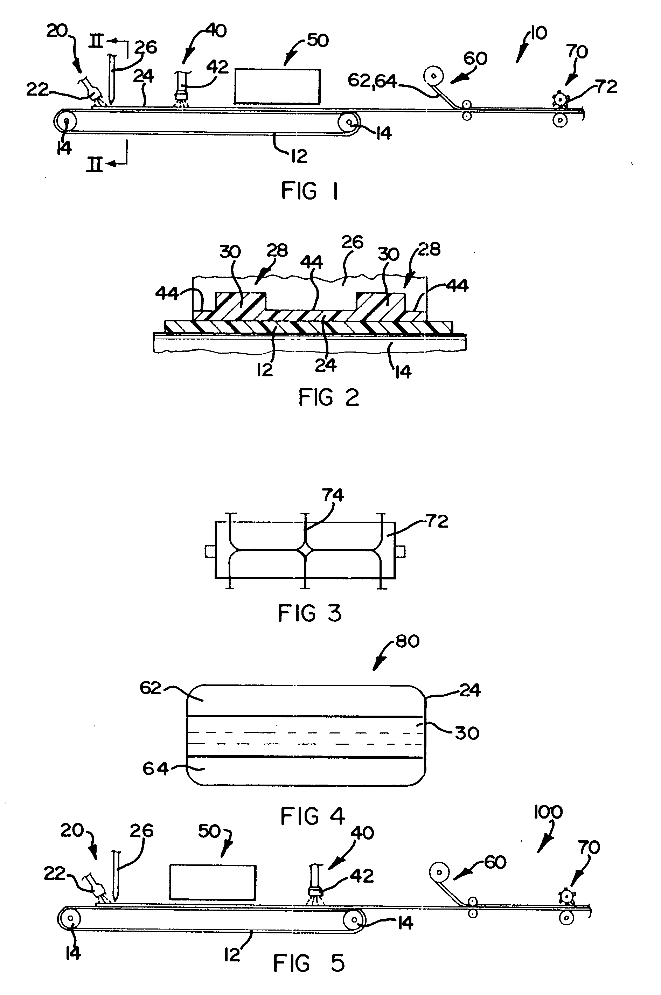

Referring to Figures 1 to 4, reference numeral 10 generally indicates a process for making an adhesive dressing, according to one embodiment of the invention.

The process 10 includes a continuous conveyer belt 12 passing over rollers 14, one of which is driven (not shown), eg by means of an electric motor/gearbox combination.

The process 10 also includes a strip forming zone, generally indicated by reference numeral 20. The strip forming zone comprises a spray applicator 22 by means of which an absorbent foam, such as polyurethane foam containing absorbency enhancing material such as acrylic superabsorbent powder, is applied as a layer or strip 24 to the upper surface of the conveyer belt 12.

A blade 26 is located immediately in front of the spray head 22. The blade 26 has recesses or grooves 28 which distribute the foam in the foam layer 24 so as to have raised areas or portions 30, the purpose of which will be described in more detail hereunder. If desired, a displaceable blade (not shown), having a straight or non-recessed edge, may be located behind the blade 26 so that instead of forming continuous raised areas 30, they can be formed intermittently by lowering and lifting the displaceable blade cyclically.

The process 10 also includes an adhesive application zone, generally indicated by reference numeral 40. In the zone 40, an adhesive spray applicator 42, for coating the foamed strip or layer 24 with a layer of curable adhesive, such as a solvent or emulsion based adhesive, is provided. The spray applicator 42 is designed such that adhesive is only applied onto the portions 44 of the foamed layer 24, adjacent the raised areas or portions 30. It will be appreciated that the foamed layer 24 passes continuously and directly through the zone 40 and through the subsequent zones as described in more detail hereunder.

The non-cured adhesive coated foamed strip 24 then passes to a curing/drying zone, generally indicated by reference numeral 50, where both the adhesive and the foam are cured at elevated temperature.

The cured strip thus formed then passes to a release paper application or laminating zone, generally indicated by reference numeral 60, where overlapping release papers 62, 64 are applied over the adhesive layers or coatings as well as over the raised areas or portions 30 which thus constitute absorbent pads.

Finally, the adhesive dressing strip thus formed passes to a cutting zone, generally indicated by reference numeral 70, where it is cut, by means of a rotating cylindrical die 72, having raised cutting edges 74 defining desired dressing shapes and sizes on its outer surface, into individual dressings of said desired shapes and sizes.

Thus, by means of the process 10, continuous dressing strips 80, as indicated in Figure 4, can be formed. The dressing strips 80 thus comprise the foamed strip or layer 24, the raised absorbent layer or pad 30, an adhesive layer on top of the foamed layer 24 adjacent the pad 30, and the release paper layers 62, 64.

Referring to Figure 5, reference numeral 100 generally indicates a process according to another embodiment of the invention, for making adhesive dressings.

In the process 100, parts which are the same or similar to those of the process 10, are indicated with the same reference numerals.

In the process 100, the adhesive application zone 40 is provided after the curing/drying zone or stage 50, and the adhesive which is applied is a hot melt adhesive applied at elevated temperature. If desired, a cooling stage (not shown) can be provided immediately after the adhesive application zone 40 to cool the adhesive to an acceptable temperature, before the release paper 62, 64 are applied thereto.

The Applicant believes that with the processes 10, 100, adhesive dressings can be made rapidly and effectively.

## Claims

1. A process for making an adhesive dressing, characterized in that it comprises
continuously feeding a strip having at least portions which are absorbent, through an adhesive application zone;
applying an adhesive to one side of the strip in such a fashion that the adhesive coated strip has at least one absorbent pad area; and
feeding the adhesive coated strip directly and continuously to a release paper application zone in which release paper is applied over the adhesive adjacent the absorbent pad area, to form a continuous adhesive dressing.

2. A process according to Claim 1, characterized in that the entire strip is absorbent, with the adhesive being applied intermittently so that the absorbent pad area is defined by non-adhesive coated areas of the strip.

3. A process according to Claim 1, characterized in that the entire strip is absorbent, with the adhesive being coated continuously over the whole strip, so that the absorbent pad area is also coated with adhesive, and with the adhesive being such that this zone will not adhere to a moist wound in use and will allow fluid absorbency, while the adhesive adjacent the absorbent pad area will adhere to dry skin adjacent such a moist wound, to secure the dressing in position.

4. A process according to any one of Claims 1 to 3 inclusive, characterized in that the absorbent strip comprises a backing layer having an absorbent layer thereon, with the process including passing the backing layer continuously through an absorbent strip forming zone ahead of the adhesive application zone, and applying the absorbent layer continuously to the backing layer in the strip forming zone.

5. A process according to Claim 4, characterized in that the absorbent layer is an absorbent fabric.

6. A process according to Claim 4, characterized in that the absorbent layer is of curable foam material, with the process including passing the strip from the strip forming zone through a curing zone ahead of the adhesive application zone, for curing of the foam material.

7. A process according to any one of Claims 1 to 3 inclusive, characterized in that the absorbent strip is of absorbent foamed material without a backing layer.

8. A process according to Claim 7, characterized in that it includes forming the absorbent foamed strip continuously in a strip forming zone ahead of the adhesive application zone.

9. A process according to Claim 8, characterized in that it includes passing the foamed strip from the strip forming zone through a curing zone where the foam is cured and stabilized, before passing it to the adhesive application zone.

10. A process according to Claim 8, characterized in that the adhesive is a curable adhesive, with the process including passing the adhesive coated strip through a curing zone located after the adhesive application zone and in which the foam and adhesive are cured.

11. A process according to any one of Claims 1 to 10 inclusive, characterized in that it includes passing the continuous adhesive dressing from the release paper application zone to a cutting zone, and cutting it into individual dressings of a desired shape and size in the cutting zone.

12. A process according to Claim 11, characterized in that the cutting is effected by means of a die cutting arrangement.
